# EUROPEAN PATENT APPLICATION

(11) **EP 4 349 443 A1**
(43) Date of publication of application: **10.04.2024**
(21) Application number: 22816115.4
(22) Date of filing: 31.05.2022
(51) Int. Cl.: B01D 9/02, C07C 51/43, C07C 57/055

(54) **HEAT EXCHANGER**

(30) Priority: 02.06.2021 JP 2021093029
(71) Applicant: Nippon Shokubai Co., Ltd., Osaka-shi, Osaka 541-0043 (JP)
(72) Inventor: SANO, Itsumi, Himeji-shi, Hyogo 671-1282 (JP); MUKAE, Masashi, Himeji-shi, Hyogo 671-1282 (JP); FUKUMOTO, Toshiya, Himeji-shi, Hyogo 671-1282 (JP)
(74) Representative: Schön, Christoph
(86) International application number: PCT/JP2022/022146
(87) International publication number: WO 2022/255369

(57) **Abstract**

The present invention provides a method for stably providing a product. The present invention relates to a heat exchanger configured to handle an easily-polymerizable compound-containing solution or an easily-polymerizable compound crystal-containing slurry, the heat exchanger including a scraper for scraping off a substance adhered to an inner wall surface thereof, a shaft connected to the scraper, and a bushing on the shaft, the bushing being to be in contact with the solution or slurry when the heat exchanger is used and being made of an aromatic polyetherketone-containing material.

## Description

### TECHNICAL FIELD

The present invention relates to a heat exchanger. Specifically, the present invention relates to a heat exchanger, a crystal-forming apparatus, a purification apparatus, a crystal-forming method, a method for producing an easily-polymerizable compound, and a method for purifying an easily-polymerizable compound.

### BACKGROUND ART

Crystal-forming apparatuses have been widely used industrially to provide high-purity products by crystallizing substances to be purified. In many fields of the chemical industry, high-quality compounds with reduced impurities have been required, and for such compounds, various better crystal-forming apparatuses have been studied.

Industrially, many of crude compounds, which are compounds before purification, are purified through continuous purification. Disclosed is a method for producing acrylic acid including: collecting and crystallization purifying a gas containing acrylic acid obtained by catalytic vapor phase oxidation of a raw material gas; and returning acrylic acid obtained by decomposing a substance obtained by Michael addition of acrylic acid in a residual mother liquor to the collecting step, for example (see, for example, Patent Literature 1).

Continuous crystal-forming apparatuses have been desired. Examples of continuous crystal-forming apparatuses include a votator heat exchanger, a scraped surface heat exchanger, and a cooling disc crystallizer.

Conventional techniques have been suggested in which in an apparatus configured to handle an easily-polymerizable liquid or an adhesive substance-containing liquid, specifically, in a pump having a shaft, parts which support the shaft (bushings) are made of fluororesin or glass-fiber reinforced fluororesin and subjected to treatment for reducing the coefficient of friction (see, for example, Patent Literature 2).

Fluororesins such as polytetrafluoroethylene (PTFE) are known to have high chemical resistance and particularly excellent frictional properties (low coefficient of friction) among resins (see, for example, Patent Literature 3) .

### CITATION LIST

### - Patent Literature

Patent Literature 1: JP 2007-182437 A
Patent Literature 2: JP H06-272688 A
Patent Literature 3: JP 2016-175955 A

### SUMMARY OF INVENTION

### - Technical Problem

The present inventors have made intensive studies on heat exchangers and crystal-forming apparatuses configured to handle easily-polymerizable compounds, and have encountered new problems as follows. In a continuous heat exchanger or crystal-forming apparatus including a shaft and a scraper for scraping off substances or crystals adhered to an inner wall surface thereof, even when a shaft bushing made of a fluororesin, which has excellent frictional properties, is used based on the conventional knowledge as described in Patent Literature 2, a polymerization reaction is unexpectedly induced by frictional heat. Thus, the polymer is caught in the sliding surface of the mechanical seal in the heat exchanger or crystal-forming apparatus, causing liquid leakage, and the heat exchanger or the crystal-forming apparatus malfunctions.

Here, since a liquid having a relatively low temperature (less polymerizable) is introduced into the heat exchanger or crystal-forming apparatus, polymerization is not expected to occur in the heat exchanger or crystal-forming apparatus.

When the heat exchanger or crystal-forming apparatus malfunctions, the operation of the heat exchanger or crystal-forming apparatus is required to temporarily stop as described in Patent Literature 2. Since stopping the operation greatly affects the whole system of the purification apparatus, it is urgently necessary to take measures to prevent or reduce the polymerization reaction caused by frictional heat and the like.

### - Solution to Problem

As a result of further intensive studies by the present inventors, they found that use of a bushing made of an aromatic polyetherketone-containing material can sufficiently prevent polymerization of the easily-polymerizable compound induced by frictional heat so that the above problems can be solved and a product can be stably obtained. The present inventors also found that the problems can be solved, without using a bushing made of a specific material, by feeding an easily-polymerizable compound-containing solution or water from the outside of the apparatus to an area around the mechanical seal installed on the shaft. Thus, the present inventors have arrived at the present invention.

That is, the present invention relates to a heat exchanger configured to handle an easily-polymerizable compound-containing solution or an easily-polymerizable compound crystal-containing slurry,
the heat exchanger including a scraper for scraping off a substance adhered to an inner wall surface thereof, a shaft connected to the scraper, and a bushing on the shaft,
the bushing being to be in contact with the solution or slurry when the heat exchanger is used and being made of an aromatic polyetherketone-containing material.

The present invention also relates to a crystal-forming apparatus configured to form easily-polymerizable compound crystals from an easily-polymerizable compound-containing solution or a mother liquor in an easily-polymerizable compound crystal-containing slurry,
the apparatus including a scraper for scraping off easily-polymerizable compound crystals formed on an inner wall surface thereof, a shaft connected to the scraper, and a bushing on the shaft,
the bushing being to be in contact with the solution or slurry when the apparatus is used and being made of an aromatic polyetherketone-containing material.

The present invention also relates to a crystal-forming method, including:
forming crystals from an easily-polymerizable compound-containing solution or a mother liquor in an easily-polymerizable compound crystal-containing slurry using a crystal-forming apparatus including a scraper for scraping off crystals formed on an inner wall surface thereof and a shaft connected to the scraper,
the apparatus further including a bushing on the shaft, the bushing being to be in contact with the solution or slurry,
the bushing being made of an aromatic polyetherketone-containing material or the method including feeding an easily-polymerizable compound-containing solution or water from the outside of the apparatus to an area around a mechanical seal installed on the shaft.

The present invention also relates to a method for producing an easily-polymerizable compound, the method including forming easily-polymerizable compound crystals from an easily-polymerizable compound-containing solution or a mother liquor in an easily-polymerizable compound crystal-containing slurry using a crystal-forming apparatus including a scraper for scraping off easily-polymerizable compound crystals formed on an inner wall surface thereof and a shaft connected to the scraper,
the apparatus further including a bushing on the shaft, the bushing being to be in contact with the solution or slurry,
the bushing being made of an aromatic polyetherketone-containing material or the method including feeding an easily-polymerizable compound-containing solution or water from the outside of the apparatus to an area around a mechanical seal installed on the shaft.

The present invention also relates to a method for purifying an easily-polymerizable compound, the method including:
forming easily-polymerizable compound crystals from an easily-polymerizable compound-containing solution or a mother liquor in an easily-polymerizable compound crystal-containing slurry using a crystal-forming apparatus including a scraper for scraping off easily-polymerizable compound crystals formed on an inner wall surface thereof and a shaft connected to the scraper,
the apparatus further including a bushing on the shaft, the bushing being to be in contact with the solution or slurry,
the bushing being made of an aromatic polyetherketone-containing material or the method including feeding an easily-polymerizable compound-containing solution or water from the outside of the apparatus to an area around a mechanical seal installed on the shaft.

PTFE, which is a typical fluororesin, is known to have better friction properties (lower coefficient of friction) than polyetheretherketone (hereinafter also referred to as PEEK), which is a typical aromatic polyetherketone (see, for example, JP 2009-236213 A). PTFE is also known to have better chemical resistance (resistance to high-concentration acid) than PEEK (see, for example, JP 2012-531024 T).

Despite the fact that an aromatic polyetherketone is known to have poorer frictional properties and poorer chemical resistance than PTFE, use of a bushing made of an aromatic polyetherketone can reduce polymerization induced by frictional heat or the like in a crystal-forming apparatus that is configured to handle high-purity, easily-polymerizable compounds, and can solve the above problems. This is an unexpected effect.

The heat exchanger of the present invention, the crystal-forming apparatus of the present invention, the crystal-forming method of the present invention, the method for producing an easily-polymerizable compound of the present invention, and the method for purifying an easily-polymerizable compound of the present invention have the same or corresponding special technical features in that they each include a shaft bushing made of an aromatic polyetherketone-containing material. At least, the heat exchanger of the present invention and the crystal-forming apparatus of the present invention have the same or corresponding special technical features in that they each include a shaft bushing made of an aromatic polyetherketone-containing material. Furthermore, at least the crystal-forming method of the present invention, the method for producing an easily-polymerizable compound of the present invention, and the method for purifying an easily-polymerizable compound of the present invention have the same or corresponding special technical features in that the shaft bushing is made of an aromatic polyetherketone-containing material or feeding an easily-polymerizable compound-containing solution or water from the outside of the apparatus to an area around a mechanical seal installed on the shaft is included.

### - Advantageous Effects of Invention

Products can be stably obtained using the heat exchanger of the present invention or the crystal-forming apparatus of the present invention.

### BRIEF DESCRIPTION OF DRAWINGS

FIG. 1 is a schematic cross-sectional side view of an exemplary crystal-forming apparatus of the present invention.
FIG. 2 is a schematic cross-sectional side view of another exemplary crystal-forming apparatus of the present invention and illustrates feeding an easily-polymerizable compound-containing solution or water from the outside of the apparatus to an area around a mechanical seal installed on a shaft.
FIG. 3(a) is a schematic cross-sectional view of an exemplary scraper, viewed from the extending direction of the shaft. FIGS. 3(b) and 3(d) are schematic cross-sectional views of other exemplary scrapers, viewed from the extending direction of the shaft. FIG. 3(c) is an enlarged view of the scraper shown in FIG. 3(b).

### DESCRIPTION OF EMBODIMENTS

The present invention will be described in detail below.

It should be noted that combinations of two or more of the preferred features of the present invention described below are also preferred embodiments of the present invention.

The following first describes the purification apparatus of the present invention, followed by descriptions of the heat exchanger of the present invention, the purification apparatus of the present invention, the method for producing an easily-polymerizable compound of the present invention, the method for purifying an easily-polymerizable compound of the present invention, and the crystal-forming method of the present invention.

Herein, the phrase "forming easily-polymerizable compound crystals" encompasses not only forming easily-polymerizable compound crystals from an easily-polymerizable compound-containing solution but also newly forming crystals from a mother liquor in an easily-polymerizable compound crystal-containing slurry and increasing the concentration of crystals in the slurry to be higher after the slurry is discharged from the apparatus than before the slurry is fed to the apparatus.

For example, the heat exchanger or crystal-forming apparatus of the present invention, the crystal-forming method of the present invention, the method for producing an easily-polymerizable compound of the present invention, and the method for purifying an easily-polymerizable compound of the present invention encompass not only those in which an easily-polymerizable compound-containing solution is fed to the crystal-forming apparatus to provide an easily-polymerizable compound crystal-containing slurry but also those in which an easily-polymerizable compound crystal-containing slurry is fed to the crystal-forming apparatus to provide a slurry having a higher crystal concentration. The easily-polymerizable compound crystal-containing slurry to be fed to the crystal-forming apparatus typically contains an easily-polymerizable compound also in its mother liquor.

When the crystals are discharged from the crystal-forming apparatus in the present invention, they are typically discharged in the form of an easily-polymerizable compound crystal-containing slurry.

### (Crystal-forming apparatus of the present invention)

The crystal-forming apparatus of the present invention is configured to form easily-polymerizable compound crystals from an easily-polymerizable compound-containing solution or a mother liquor in an easily-polymerizable compound crystal-containing slurry, the apparatus including a scraper for scraping off easily-polymerizable compound crystals formed on an inner wall surface thereof, a shaft connected to the scraper, and a bushing on the shaft, the bushing being to be in contact with the solution or slurry when the apparatus is used and being made of an aromatic polyetherketone-containing material. The bushing is configured to prevent vibration of the shaft.

The bushing made of an aromatic polyetherketone-containing material can sufficiently prevent polymerization of the easily-polymerizable compound induced by frictional heat between the bushing and the shaft. This can result in sufficient prevention of the displacement of the mechanical seal sliding surface caused by the polymer and the accompanying liquid leakage. Thereby, the crystal-forming apparatus can be sufficiently prevented from malfunctioning, and can stably operate for a long period of time. As a result, the productivity can be improved and the maintenance costs can be reduced.

As described above, although polyetheretherketone, which is a typical aromatic polyetherketone, has a higher coefficient of friction than PTFE, use of a bushing made of an aromatic polyetherketone-containing material can reduce polymerization induced by frictional heat. This is an unexpected effect.

The aromatic polyetherketone is a polymer having a structural unit containing an optionally substituted divalent aromatic group (preferably a phenylene group), an ether group, and a ketone group (a carbonyl group). Examples of the aromatic polyetherketone include polyetheretherketone (PEEK), polyetherketone (PEK), polyetherketone ketone (PEKK), polyetheretherketone ketone (PEEKK), and polyetherketone ether ketone ketone (PEKEKK). One or more of these may be used.

As for the aromatic polyetherketone, the mass percentage of the structural unit containing an optionally substituted divalent aromatic group, an ether group, and a ketone group in 100% by mass of the aromatic polyetherketone is preferably 50% by mass or more, more preferably 60% by mass or more, still more preferably 70% by mass or more, further more preferably 80% by mass or more, particularly preferably 90% by mass or more.

The upper limit of the mass percentage of the structural unit is not limited, and may be 100% by mass, and is most preferably 100% by mass.

In particular, the aromatic polyetherketone is preferably polyetheretherketone.

Herein, polyetheretherketone is a polymer having a structural unit in which an ether group, an ether group, and a ketone group as functional groups are arranged in this order with optionally substituted divalent aromatic groups being present therebetween. For example, it is preferably a polymer having a structural unit represented by the following formula (1).

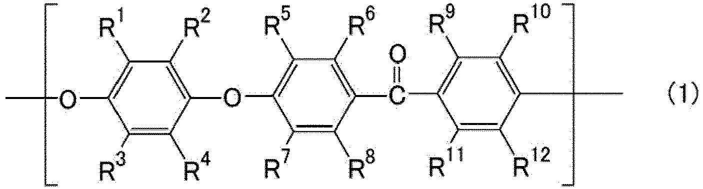

In the formula (1), R¹ to R¹² are the same as or different from each other and each represent a hydrogen atom or a hydrocarbon group. Adjacent hydrocarbon groups may bond together to form a ring structure.

Suitable examples of the hydrocarbon group include C1-C12 aliphatic hydrocarbon groups and C6-C12 aromatic hydrocarbon groups.

As for the polyetheretherketone, the mass percentage of the structural unit represented by the formula (1) in 100% by mass of the polyetheretherketone is preferably 50% by mass or more, more preferably 60% by mass or more, still more preferably 70% by mass or more, further more preferably 80% by mass or more, particularly preferably 90% by mass or more.

The upper limit of the mass percentage of the structure represented by the formula (1) is not limited, and may be 100% by mass, and is most preferably 100% by mass.

In particular, R¹ to R¹² each preferably represent a hydrogen atom.

In other words, the polyetheretherketone is preferably a polymer having a structural unit represented by the following formula (2).

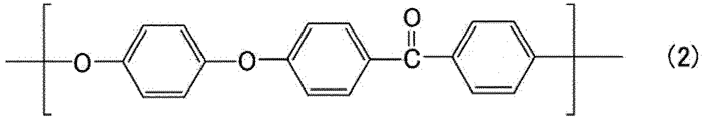

As for the polyetheretherketone, the mass percentage of the structural unit represented by the formula (2) in 100% by mass of the polyetheretherketone is preferably 50% by mass or more, more preferably 60% by mass or more, still more preferably 70% by mass or more, further more preferably 80% by mass or more, particularly preferably 90% by mass or more.

The upper limit of the mass percentage of the structural unit represented by the formula (2) is not limited, and may be 100% by mass, and is most preferably 100% by mass.

As for the aromatic polyetherketone-containing material, the mass percentage of the aromatic polyetherketone in 100% by mass of the aromatic polyetherketone-containing material is preferably 30% by mass or more, more preferably 50% by mass or more, still more preferably 80% by mass or more, particularly preferably 100% by mass.

The aromatic polyetherketone-containing material for the bushing may contain a different component such as resin (e.g., thermoplastic resin) or an additive (e.g., a pigment). The bushing is preferably made of an aromatic polyetherketone (more preferably polyetheretherketone), as described above.

The bushing can be obtained, for example, by molding an aromatic polyetherketone-containing material. The molding may be performed by an appropriate known method for molding a resin composition.

The bushing may have a ring shape or a cylindrical shape.

The bushing is at least partly in contact with the solution or slurry when the apparatus is used (properly). The bushing should be in contact with the solution or crystal-containing slurry when the apparatus is used, and may not be in contact with the solution or crystal-containing slurry when the apparatus is not used. In other words, the apparatus of the present invention is not limited to an apparatus in use.

The length of the bushing (the length in the longitudinal direction of the shaft) cannot be generalized, and is typically about 10 to 200 mm.

The difference between the inner diameter of the bushing and the outer diameter of the shaft cannot be generalized, and is typically 0.01 mm or more. With such a structure, the frictional heat generated between the shaft and the bushing can be reduced, and the polymerization can be prevented more sufficiently.

Preferably, the difference between the inner diameter of the bushing and the outer diameter of the shaft is 1 mm or less. With such a structure, vibration of the shaft can be sufficiently prevented.

The number of bushings in the crystal-forming apparatus of the present invention is not limited, and may be appropriately selected according to the size of the apparatus.

When the crystal-forming apparatus of the present invention includes multiple bushings, at least one of the bushings should be in contact with the solution or crystal-containing slurry when the apparatus is used. For example, when multiple bushings are arranged on the shaft, at least one of the bushings should be in contact with the solution or crystal-containing slurry.

When the apparatus of the present invention includes multiple bushing, preferably, adjacent bushings are spaced at a distance of 5000 mm or less.

The distance between adjacent bushings is more preferably 4500 mm or less, still more preferably 4000 mm or less.

The distance between adjacent bushings is more preferably 100 mm or more, still more preferably 300 mm or more, particularly preferably 1000 mm or more.

When multiple spaces are present among the bushings, at least one space between adjacent bushings may have a distance within the above range. Preferably, all of the spaces each have a distance within the above range.

The scraper for scraping off the easily-polymerizable compound crystals formed on the inner wall surface of the crystal-forming apparatus (typically, the inner wall surface of the crystal-forming section) may be made of any material and is preferably made of a material containing a resin. In particular, in the crystal-forming apparatus of the present invention, the scraper is preferably made of an aromatic polyetherketone-containing material.

The scraper may be entirely made of an aromatic polyetherketone-containing material, or part of the scraper (e.g., part proximate to the inner wall surface of the crystal-forming apparatus) may be made of an aromatic polyetherketone-containing material, for example. Thereby, heat generation due to friction between the scraper and the inner wall surface of the crystal-forming apparatus is reduced. As a result, polymerization of the easily-polymerizable compound is prevented, for example, and more stable production of a product can be expected.

Preferred types and preferred amounts of the aromatic polyetherketone in the material of the scraper are the same as the preferred types and preferred amounts of the aromatic polyetherketone described above as the material of the bushing. For example, the aromatic polyetherketone in the material of the scraper is preferably polyetheretherketone.

In other words, in the crystal-forming apparatus of the present invention, the bushing and/or scraper is preferably made of a polyetheretherketone-containing material.

The distance from the central axis of the shaft to the part of the scraper closest to the inner wall surface may be appropriately selected according to the inner diameter of the crystal-forming apparatus of the present invention (usually, the crystal-forming section). In order to scrape off the crystals formed and adhered to the inner wall surface of the crystal-forming apparatus, the scraper is preferably in contact with the inner wall surface.

The scraper may have any shape, and typically has a substantially trapezoidal thin plate shape or a substantially rectangular plate shape. For example, the scraper preferably has a substantially rectangular plate shape, with the side proximate to the inner wall surface of the crystal-forming apparatus being the longer side.

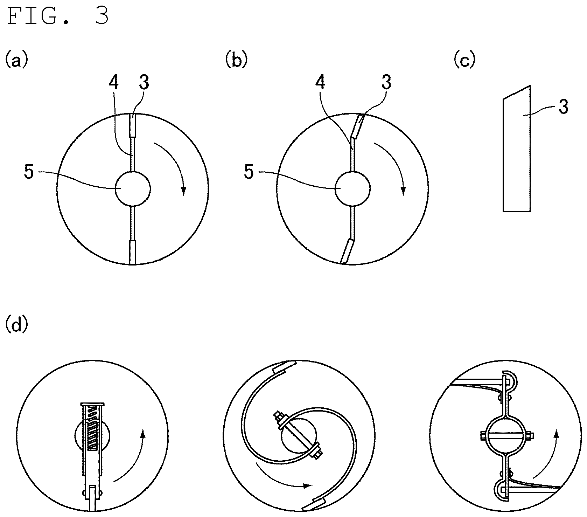

The substantially rectangular plate shape is, for example, a rectangular plate shape as shown in FIG. 3(a) having a flat lateral face in a part proximate to the inner wall surface of the crystal-forming apparatus. Also, as shown in FIGS. 3(b) and 3(c), the substantially rectangular plate shape may have a blade-like part with an acute angle in a part proximate to the inner wall surface of the crystal-forming apparatus. Similarly, the substantially trapezoidal plate shape may have a flat lateral face or a blade-like part with an acute angle in a part proximate to the inner wall surface of the crystal-forming apparatus.

As described above, the scraper may have an inclined edge like a blade, and scrape off crystals with the edge.

Further, the scraper and a connection, which is described later, may be joined in a straight line as shown in FIG. 3(a). The scraper may be joined at an angle to the connection as shown in FIG. 3(b).

The crystal-forming apparatus of the present invention may include one scraper or multiple scrapers. For example, multiple scrapers may be arranged along the rotational direction of the shaft, or multiple scrapers may be arranged along the longitudinal direction of the shaft.

The crystal-forming apparatus of the present invention may further include a connection connecting the scraper and the shaft.

The connection may be made of any material and may have any shape as long as it can connect the scraper and the shaft. The connection may be made of a metal such as stainless steel or a resin, for example. Also, the connection may be integrated with the scraper.

The connection is preferably attached to the shaft so as to make an angle of 60° or more and 120° or less, more preferably a right angle with respect to the longitudinal direction of the shaft.

The shape and number of the connections are not limited as long as the connection connects the scraper and the shaft.

The shaft may be directly connected to the scraper, or may be connected to the scraper via the connection. Preferably, the shaft is connected to the scraper via the connection.

The shaft may be made of any material and may be made of a metal such as stainless steel or a resin. The shaft is preferably made of a metal, more preferably made of stainless steel (SUS).

The shaft may be a single shaft consisting of multiple shafts connected to each other in the longitudinal direction of the shaft.

Preferably, the longitudinal direction of the shaft is in a horizontal direction (horizontal type).

In the crystal-forming apparatus of the present invention, the easily-polymerizable compound is preferably a (meth)acrylic acid. In other words, the crystal-forming apparatus of the present invention preferably forms (meth)acrylic acid crystals from a (meth)acrylic acid-containing solution or a mother liquor in a slurry containing (meth)acrylic acid crystals. Here, the term "(meth)acrylic acid" refers to acrylic acid and/or methacrylic acid.

The mass percentage of the easily-polymerizable compound in the easily-polymerizable compound-containing solution or the mother liquid in the easily-polymerizable compound crystal-containing slurry is not limited and may be low. Preferably, the mass percentage thereof falls within the preferred range described later in the production method of an easily-polymerizable compound of the present invention.

In the crystal-forming apparatus of the present invention, the crystal-forming section includes a feed port for an easily-polymerizable compound-containing solution or an easily-polymerizable compound crystal-containing slurry (hereinafter also simply referred to as a feed port for an easily-polymerizable compound) and a discharge port for an easily-polymerizable compound crystal-containing slurry. The directions of the feed port and the discharge port for an easily-polymerizable compound are not limited. For example, the crystal-forming apparatus of the present invention may include the feed port for an easily-polymerizable compound in a lower part of the crystal-forming section and the discharge port for an easy-polymerizable compound crystal-containing slurry in an upper part thereof.

The crystal-forming apparatus of the present invention should include one feed port for an easily-polymerizable compound and one discharge port for an easily-polymerizable compound crystal-containing slurry and may include two or more of each of these.

The crystal-forming apparatus of the present invention may have any size, and preferably has an inner diameter (the inner diameter of the crystal-forming section) of 100 to 500 mm. The crystal-forming section preferably has a lateral length of 2000 to 20000 mm.

The crystal-forming apparatus of the present invention typically further includes a mechanism for cooling the whole or part of the inner wall surface thereof in order to form crystals from an easily-polymerizable compound-containing solution or a mother liquor in an easily-polymerizable compound crystal-containing slurry.

In particular, the crystal-forming apparatus of the present invention preferably includes a cooling-medium feed port and a cooling-medium discharge port. A cooling medium can suitably cool the inner wall surface of the crystal-forming section, suitably forming crystals.

The directions of the cooling-medium feed port and the cooling-medium discharge port are not limited. For example, the cooling-medium feed port may be located in a lower part of the crystal-forming section and the cooling-medium discharge port may be located in an upper part of the crystal-forming section.

The crystal-forming apparatus of the present invention should include one cooling-medium feed port and one cooling-medium discharge port and may include two or more of each of these.

Non-limiting examples of the cooling medium include any liquids and gases such as water, antifreeze, a methanol water mixture (an aqueous methanol solution), and gas. The heating medium may be appropriately selected in consideration of the freezing point of the compound to be purified, for example.

The crystal-forming apparatus of the present invention preferably includes a feed port for feeding an easily-polymerizable compound-containing solution or water from the outside of the apparatus to an area around the mechanical seal installed on the shaft. The easily-polymerizable compound-containing solution to be used typically has a melting point lower than that of the solution or the mother liquor in the slurry in the crystal-forming section. The melting point of water is typically lower than the melting point of the solution or the mother liquor in the slurry in the crystal-forming section. Thereby, the easily-polymerizable compound-containing solution or water after feeding can be prevented from freezing, the solution or slurry derived from the crystal-forming section can be sufficiently prevented from retaining in an area around the mechanical seal, and the displacement of the sliding surface of the mechanical seal caused by the polymer and liquid leakage can be further prevented.

Herein, an area around the mechanical seal refers to a gap formed between the mechanical seal and the bushing.

The crystal-forming apparatus of the present invention should include one feed port and may have multiple feed ports.

Preferably, the distance between the mechanical seal and the bushing is 100 mm or less. With such a structure, the amount of liquid or slurry retained between the mechanical seal and the bushing can be reduced. The distance between the mechanical seal and the bushing is more preferably 80 mm or less, still more preferably 60 mm or less.

Instruments such as a thermometer, a pressure gauge, a liquid level gauge (e.g., of radar type), and a level switch (e.g., of float type) may be provided in the main body or periphery of the crystal-forming apparatus of the present invention. The crystal-forming apparatus may include a sight glass (an observation window) in its side plate or the like. The sight glass may be covered with a cover. The crystal-forming apparatus may include a hand hole (a hole for inserting a hand for maintenance) and a safety valve in its top plate, side plate, or the like. The numbers of these are not limited.

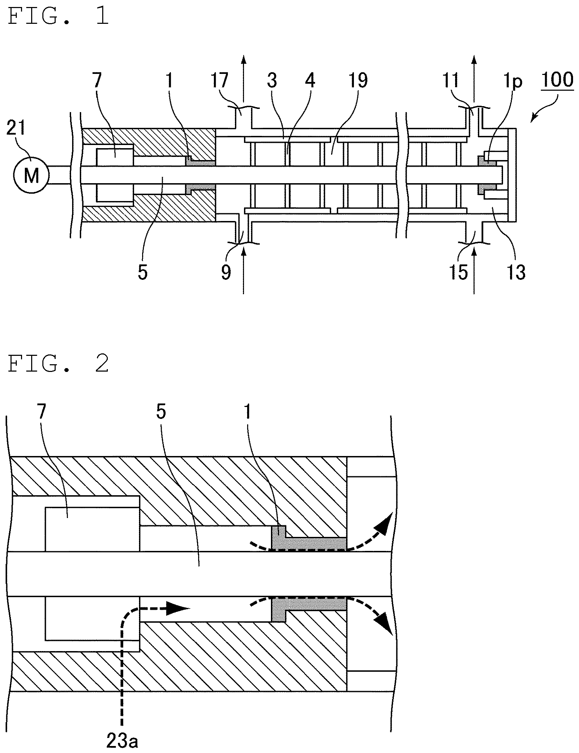

FIG. 1 is a schematic cross-sectional side view of an exemplary crystal-forming apparatus of the present invention. An easily-polymerizable compound such as a (meth)acrylic acid is fed to a crystal-forming section 19 of a crystal-forming apparatus 100 through an easily-polymerizable compound feed port 9. A cooling medium is flown around the crystal-forming section 19 through a cooling-medium feed port 15 and a cooling-medium discharge port 17, and whereby easily-polymerizable compound crystals are formed on the cooled inner wall surface. Next, a shaft 5 is rotated by a power unit (motor) 21 of the crystal-forming apparatus 100 to scrape off the crystals adhered to the inner wall surface by a scraper 3 connected to the shaft 5 via a connection 4. Thus, a crystal-containing slurry 13 is obtained in the crystal-forming section 19. The crystal-containing slurry 13 is in contact with bushings 1 and 1p on the shaft 5. The bushings 1 and 1p are made of an aromatic polyetherketone-containing material, and whereby polymerization of the easily-polymerizable compound caused by frictional heat can be sufficiently prevented. The easily-polymerizable compound crystal-containing slurry is discharged from a discharge port 11 for an easily-polymerizable compound crystal-containing slurry. In the case of continuous purification, the steps are typically performed simultaneously when the crystal-forming apparatus is viewed as a whole.

As described above, the crystal-forming apparatus shown in FIG. 1 can sufficiently prevent polymerization of the easily-polymerizable compound caused by frictional heat, so that the displacement of the sliding surface of a mechanical seal 7 caused by the polymer and liquid leakage can be sufficiently prevented.

FIG. 2 is a schematic cross-sectional side view of another exemplary crystal-forming apparatus of the present invention. In the crystal-forming apparatus of the present invention shown in FIG. 2, an easily-polymerizable compound-containing solution or water 23a is fed to an area around the mechanical seal 7 installed on the shaft 5. The easily-polymerizable compound-containing solution or water 23a after feeding flows between the bushing 1 and the shaft 5 and is discharged to the crystal-forming section. Thereby, the solution or slurry derived from the crystal-forming section can be sufficiently prevented from retaining in an area around the mechanical seal 7, and the displacement of the sliding surface of the mechanical seal 7 caused by the polymer and liquid leakage can be further prevented.

### (Heat exchanger of the present invention)

The heat exchanger of the present invention is configured to handle an easily-polymerizable compound-containing solution or an easily-polymerizable compound crystal-containing slurry, the heat exchanger including a scraper for scraping off a substance adhered to an inner wall surface thereof, a shaft connected to the scraper, and a bushing on the shaft, the bushing being to be in contact with the solution or slurry when the heat exchanger is used and being made of an aromatic polyetherketone-containing material. The scraper, shaft, and bushing in the heat exchanger of the present invention are the same as the scraper, shaft, and bushing in the above-described crystal-forming apparatus of the present invention. The mass percentage of the easily-polymerizable compound in the easily-polymerizable compound-containing solution or the mother liquid in the easily-polymerizable compound crystal-containing slurry is not limited and may be low. The substance adhered to the inner wall surface may be easily-polymerizable compound crystals and/or a non-crystalline easily-polymerizable compound, or may be a component other than the easily-polymerizable compound. Also, the substance adhered to the inner wall surface may be a target substance or impurities. Since the crystal-forming apparatus of the present invention typically forms crystals on the inner wall surface thereof through cooling by heat exchange with a cooling medium, the crystal-forming apparatus of the present invention can be said to be a type of heat exchanger or a heat exchanger that forms easily-polymerizable compound crystals.

### (Purification apparatus of the present invention)

The present invention also relates to a purification apparatus including the heat exchanger or crystal-forming apparatus of the present invention.

Preferably, the purification apparatus of the present invention is capable of performing continuous purification.

In the purification apparatus of the present invention, crystals formed in the heat exchanger or the crystal-forming apparatus of the present invention are subjected to solid-liquid separation to obtain purified crystals. For example, a wash column is preferably used as a solid-liquid separator. If necessary, a tank, a pump, or the like may be provided in the preceding stage and/or the subsequent stage of the heat exchanger or crystal-forming apparatus of the present invention.

The purification apparatus of the present invention may further include a mechanism that controls the amount of the slurry to be sent and the amount of the mother liquor to be returned. Examples of the control mechanism include valves attached to the lines.

The purification apparatus of the present invention may appropriately include different units commonly used in the purification apparatus.

### (Method for producing easily-polymerizable compound of the present invention)

The present invention also relates to a method for producing an easily-polymerizable compound, the method including forming easily-polymerizable compound crystals from an easily-polymerizable compound-containing solution or a mother liquor in an easily-polymerizable compound crystal-containing slurry using a crystal-forming apparatus including a scraper for scraping off easily-polymerizable compound crystals formed on an inner wall surface thereof and a shaft connected to the scraper, the apparatus further including a bushing on the shaft, the bushing being to be in contact with the solution or slurry, the bushing being made of an aromatic polyetherketone-containing material or the method including feeding an easily-polymerizable compound-containing solution or water from the outside of the apparatus to an area around a mechanical seal installed on the shaft.

As for the method for producing an easily-polymerizable compound of the present invention, the following describes forming easily-polymerizable compound crystals, followed by descriptions of other steps. In the case of continuous purification, the steps are typically performed simultaneously when the crystal-forming apparatus is viewed as a whole.

Herein, the term "easily-polymerizable compound" refers to an easily-polymerizable compound obtainable by the production method of the present invention, and does not refer to raw materials, by-products, and solvents in the production method of the present invention. The term "easily-polymerizable compound" may also be referred to as a "polymerizable compound", a "target compound" or a "target substance". Herein, the "impurities" refer to components other than the "easily-polymerizable compound" obtainable by the production method of the present invention, such as raw materials, by-products, and solvents. The crystal-forming apparatus of the present invention is also effective when polymerizable raw materials, polymerizable by-products, or polymerizable solvents are used.

### <Forming easily-polymerizable compound crystals>

The production method of the present invention includes forming easily-polymerizable compound crystals from an easily-polymerizable compound-containing solution or a mother liquor in an easily-polymerizable compound crystal-containing slurry using the crystal-forming apparatus of the present invention.

In the apparatus of the present invention, the temperature of the crystal-forming section that is configured to form easily-polymerizable compound crystals from an easily-polymerizable compound-containing solution or a mother liquor in an easily-polymerizable compound crystal-containing slurry may be controlled depending on the type of the easily-polymerizable compound. The temperature of the crystal-forming section is approximately preferably -1°C to -15°C, more preferably -1.5°C to - 13.5°C, still more preferably -3.5°C to -12.5°C, particularly preferably -5°C to -11.5°C from the melting point of the pure substance. Thereby, the crystals can be suitably formed.

When the easily-polymerizable compound is a (meth)acrylic acid, the temperature is preferably 0°C to 12°C, more preferably 1°C to 10°C, still more preferably 2°C to 8.5°C.

The temperature of the crystal-forming section can be determined by measuring the temperature of the slurry in the apparatus or in the discharge port (pipe) with a thermometer.

The feed temperature of the cooling medium to be fed to the crystal-forming apparatus of the present invention is not limited and may be appropriately selected in consideration of the freezing point of the compound to be purified and the like. Preferably, the feed temperature is lower than the temperature of the crystal-forming section by 3°C to 30°C. In other words, the feed temperature of the cooling medium is lower than the temperature of the crystal-forming section preferably by 3°C or more, more preferably by 5°C or more. The feed temperature is preferably within 30°C, more preferably within 25°C, still more preferably within 20°C with respect to the temperature of the crystal-forming section.

The amount of the cooling medium fed to the crystal-forming apparatus of the present invention is not limited and may be appropriately selected depending on the amount of formed crystals, the pressure loss from the cooling-medium feed port to the cooling-medium discharge port, and the like.

Preferably, in the forming crystals, the shaft is rotated to rotate the scraper connected to the shaft in the crystal-forming section so that the crystals adhered to the inner wall of the crystal-forming section are scraped off.

Typically, the shaft may be rotated using a motor or the like.

Preferably, in the forming crystals, the shaft is rotated at a rotation speed of 1 to 80 rpm.

The rotation speed of the shaft is more preferably 10 rpm or more, still more preferably 20 rpm or more. The rotation speed of the shaft is more preferably 60 rpm or less, still more preferably 50 rpm or less.

Preferably, in the forming crystals, the shaft is rotated at a peripheral speed of 0.02 to 1.2 m/s. The peripheral speed of the shaft is more preferably 0.15 m/s or more, still more preferably 0.3 m/s or more. The peripheral speed is more preferably 0.9 m/s or less, still more preferably 0.8 m/s or less.

The rotation of the shaft may be intermittent, or when the crystal-forming apparatus of the present invention is used, the rotation may be basically continuous. Regardless of whether the rotation of the shaft is intermittent or continuous, the rotation speed during rotation preferably falls within the above-described preferred range.

In the production method of the present invention, the easily-polymerizable compound is preferably an easily-polymerizable compound having a reactive double bond.

In particular, in the production method of the present invention, the easily-polymerizable compound is more preferably an unsaturated carboxylic acid, still more preferably a (meth)acrylic acid, particularly preferably acrylic acid. Herein, the term "(meth)acrylic acid" refers to acrylic acid and/or methacrylic acid.

### <Feeding easily-polymerizable compound-containing solution or easily-polymerizable compound crystal-containing slurry to crystal-forming apparatus>

The production method of the present invention preferably includes feeding an easily-polymerizable compound-containing solution or an easily-polymerizable compound crystal-containing slurry to the crystal-forming apparatus.

In the feeding, the easily-polymerizable compound-containing solution or the easily-polymerizable compound crystal-containing slurry is typically fed from the outside of the crystal-forming apparatus to the crystal-forming section of the crystal-forming apparatus. The easily-polymerizable compound-containing solution or the easily-polymerizable compound crystal-containing slurry may be prepared, for example, using an apparatus provided in the preceding stage of the crystal-forming apparatus.

In the feeding, the feed rate of the easily-polymerizable compound-containing solution or the easily-polymerizable compound crystal-containing slurry is not limited. In an industrial-scale crystal-forming apparatus, the feed rate is 10 to 1000 t/h, for example.

In the feeding, the feed temperature of the easily-polymerizable compound-containing solution or the easily-polymerizable compound crystal-containing slurry may be appropriately adjusted within the range of 0°C to 25°C, for example.

For example, when the easily-polymerizable compound is a (meth)acrylic acid, the feed temperature of the easily-polymerizable compound-containing solution or the easily-polymerizable compound crystal-containing slurry is preferably 1°C to 10°C, more preferably 2°C to 8.5°C.

Examples of the easily-polymerizable compound-containing solution or easily-polymerizable compound crystal-containing slurry include those containing the easily-polymerizable compound, an aqueous solution of the easily-polymerizable compound, and an aqueous slurry containing crystals of the easily-polymerizable compound. The easily-polymerizable compound-containing solution or the mother liquor in the easily-polymerizable compound crystal-containing slurry typically contain impurities other than the easily-polymerizable compound, and the aqueous solution of the easily-polymerizable compound or the aqueous slurry containing crystals of the easily-polymerizable compound typically contains impurities other than the easily-polymerizable compound and water.

In the method for producing an easily-polymerizable compound of the present invention, the purity (mass percentage) of the easily-polymerizable compound in the easily-polymerizable compound-containing solution or the mother liquor in the easily-polymerizable compound crystal-containing slurry is preferably 99% by mass or lower. The purity is preferably equal to or higher than the eutectic point with impurities.

When the easily-polymerizable compound is a (meth)acrylic acid, the purity (mass percentage) thereof is preferably 99% by mass or less, more preferably 98.5% by mass or less, still more preferably 95% by mass or less. The purity is preferably 80% by mass or more, more preferably 85% by mass or more.

The mass percentage of the easily-polymerizable compound in the easily-polymerizable compound-containing solution or the mother liquor in the easily-polymerizable compound crystal-containing slurry is preferably 75% by mass or more.

The mass percentage of the easily-polymerizable compound in the easily-polymerizable compound crystal-containing slurry refers to the mass percentage of the easily-polymerizable compound in the mother liquor thereof.

The easily-polymerizable compound-containing solution or the easily-polymerizable compound crystal-containing slurry refers to the easily-polymerizable compound-containing solution or the easily-polymerizable compound crystal-containing slurry immediately before being fed to the crystal-forming apparatus (e.g., the easily-polymerizable compound-containing solution or the easily-polymerizable compound crystal-containing slurry in a pipe or nozzle that feeds the easily-polymerizable compound-containing solution or the easily-polymerizable compound crystal-containing slurry to the crystal-forming apparatus).

Typically, accompanying the feeding, the easily-polymerizable compound crystal-containing slurry is discharged from the crystal-forming apparatus.

The crystal-containing slurry discharged from the crystal-forming apparatus is a suspension of easily-polymerizable compound crystals and a mother liquor. In other words, the liquid portion of the easily-polymerizable compound crystal-containing slurry is the mother liquor. The easily-polymerizable compound-containing solution or the easily-polymerizable compound crystal-containing slurry to be fed to the crystal-forming apparatus may be prepared in-house or procured from outside sources. The easily-polymerizable compound-containing solution or the easily-polymerizable compound crystal-containing slurry herein encompasses crude compounds.

The mass percentage of the crystals in the crystal-containing slurry discharged from the crystal-forming apparatus is preferably 1% by mass or more, more preferably 5% by mass or more, still more preferably 10% by mass or more, particularly preferably 15% by mass or more.

The mass percentage of the crystals is preferably 40% by mass or less, more preferably 38% by mass or less, still more preferably 35% by mass or less.

The crystal-containing slurry discharged from the crystal-forming apparatus preferably contains the easily-polymerizable compound in the mother liquor. Examples of the mother liquor include the easily-polymerizable compound and the aqueous solution of the easily-polymerizable compound. The mother liquor typically contains impurities other than the easily-polymerizable compound, and the aqueous solution of the easily-polymerizable compound or the aqueous slurry containing crystals of the easily-polymerizable compound typically contain impurities other than the easily-polymerizable compound and water.

In the method for producing an easily-polymerizable compound of the present invention, the purity (mass percentage) of the easily-polymerizable compound in the mother liquor in the crystal-containing slurry discharged from the crystal-forming apparatus is preferably 99% by mass or less. The purity is preferably equal to or higher than the eutectic point with impurities. When the easily-polymerizable compound is a (meth)acrylic acid, the purity (mass percentage) thereof is preferably 99% by mass or less, more preferably 98.5% by mass or less, still more preferably 95% by mass or less. The purity is preferably 80% by mass or more, more preferably 85% by mass or more.

The mass percentage of the easily-polymerizable compound in the mother liquor is preferably 70% by mass or more.

The mass percentage of water in the mother liquor is preferably 1% by mass or more. The mass percentage of water is preferably 10% by mass or less.

The crystal-containing slurry may be discharged at any discharge rate from the crystal-forming apparatus. For example, the discharge rate is 10 to 1000 t/h in an industrial-scale crystal-forming apparatus.

### <Feeding easily-polymerizable compound-containing solution or water from outside of crystal-forming apparatus to periphery of mechanical seal installed on shaft>

The production method of the present invention preferably further includes feeding an easily-polymerizable compound-containing solution or water from the outside of the apparatus to an area around a mechanical seal installed on the shaft.

In the method for producing an easily-polymerizable compound of the present invention, in order to prevent freezing of the easily-polymerizable compound-containing solution or water used in the feeding, preferably, in the crystal-forming section configured to form easily-polymerizable compound crystals from an easily-polymerizable compound-containing solution or a mother liquor in an easily-polymerizable compound crystal-containing slurry in the apparatus, the solution or the mother liquor in the slurry has a melting point higher than the melting point of the easily-polymerizable compound-containing solution or water used in the feeding.

For example, the melting point of the solution or the mother liquor in the slurry in the crystal-forming section is higher than the melting point of the easily-polymerizable compound-containing solution or water used in the feeding more preferably by 1°C or more, still more preferably by 2°C or more, particularly preferably by 3°C or more.

The upper limit of the difference between the melting point of the solution or the mother liquid in the slurry in the crystal-forming section and the melting point of the easily-polymerizable compound-containing solution or water used in the feeding is not limited, and is typically 10°C or less.

In the feeding, the easily-polymerizable compound-containing solution or water is typically fed from the outside of the apparatus.

In the feeding, the feed rate of the easily-polymerizable compound-containing solution or water should be sufficient to prevent retention of the solution or slurry derived from the crystal forming section in an area around the mechanical seal and may be appropriately selected.

The feeding may be intermittent, or when the crystal-forming apparatus of the present invention is used, the feeding may be continuous.

In the feeding, the feed temperature of the easily-polymerizable compound-containing solution or water can be appropriately adjusted within the range of higher than 0°C and not higher than 40°C, for example.

For example, when the easily-polymerizable compound is a (meth)acrylic acid, the feed temperature of the easily-polymerizable compound-containing solution or water is preferably 3°C to 12°C, more preferably 4°C to 10°C.

The feed temperature of the easily-polymerizable compound-containing solution or water is the temperature of the easily-polymerizable compound-containing solution or water immediately before being fed to the crystal-forming apparatus (e.g., the easily-polymerizable compound-containing solution or water in a pipe or nozzle that feeds the easily-polymerizable compound-containing solution or water to the crystal-forming apparatus).

The crystal-forming apparatus may be operated with its interior being under increased pressure, atmospheric pressure, or reduced pressure.

In the feeding, any amount of the easily-polymerizable compound-containing solution or water may be fed. Since the pressure at an area around the mechanical seal 7 is preferably higher than the pressure in the crystal-forming apparatus, and is more preferably constantly kept at a pressure higher than the pressure in the crystal-forming apparatus, the easily-polymerizable compound-containing solution or water is preferably fed in an amount that achieves these pressure conditions. For example, the amount of the solution or water is preferably 0.01 to 5.0 L/h, more preferably 0.1 to 3.0 L/h.

### <Obtaining easily-polymerizable compound-containing solution or easily-polymerizable compound crystal-containing slurry from raw material>

In the production method of the present invention, the easily-polymerizable compound-containing solution or the easily-polymerizable compound crystal-containing slurry may be prepared in-house or procured from outside sources, as described above. Preferably, the production method of the present invention further includes obtaining the easily-polymerizable compound-containing solution or the easily-polymerizable compound crystal-containing slurry from a raw material.

The obtaining an easily-polymerizable compound-containing solution or an easily-polymerizable compound crystal-containing slurry is not limited as long as an easily-polymerizable compound-containing solution or an easily-polymerizable compound crystal-containing slurry can be obtained. For example, the easily-polymerizable compound-containing solution or the easily-polymerizable compound crystal-containing slurry can be obtained by collecting an easily-polymerizable compound gas, which is a reaction product obtained in a reactor, in an absorption column. The easily-polymerizable compound-containing solution or the easily-polymerizable compound crystal-containing slurry encompasses a crude compound obtained by partial purification of the collected substance. The easily-polymerizable compound-containing solution or the easily-polymerizable compound crystal-containing slurry also encompasses the collected substance and a mother liquor in the crystal-containing slurry to be discharged obtained by feeding the collected substance to the crystal-forming apparatus of the present invention. When the easily-polymerizable compound is a (meth)acrylic acid, the obtaining an easily-polymerizable compound-containing solution or an easily-polymerizable compound crystal-containing slurry can be suitably performed by, for example, the method described in JP 2007-182437 A (Patent Literature 1) including synthesizing acrylic acid, collecting the acrylic acid, and optional crystallizing.

Preferably, in the method for producing an easily-polymerizable compound of the present invention, the (meth)acrylic acid is produced from at least one raw material selected from the group consisting of propane, propylene, acrolein, isobutene, methacrolein, acetic acid, lactic acid, isopropanol, 1,3-propanediol, glycerol, and 3-hydroxypropionic acid.

In the obtaining an easily-polymerizable compound-containing solution or an easily-polymerizable compound crystal-containing slurry, basically, impurities such as by-products are generated, and the easily-polymerizable compound-containing solution or the easily-polymerizable compound crystal-containing slurry contains impurities other than the easily-polymerizable compound. The aqueous solution of the easily-polymerizable compound or the aqueous slurry containing crystals of the easily-polymerizable compound typically contains impurities other than the easily polymerizable compound and water.

For example, when the easily-polymerizable compound is a (meth)acrylic acid, the impurities generated include water; acids such as propionic acid, acetic acid, maleic acid, benzoic acid, and acrylic acid dimers; aldehydes such as acrolein, furfural, formaldehyde, and glyoxal; acetone; methyl isobutyl ketone; toluene; and protoanemonin. Yet, purification or the like by the crystal-forming apparatus in the production method of the present invention can efficiently provide a high-quality product.

In the production method of the present invention, the easily-polymerizable compound-containing solution is preferably a crude (meth)acrylic acid aqueous solution or a crude (meth)acrylic acid solution.

The crude (meth)acrylic acid aqueous solution refers to a solution in which a (meth)acrylic acid is dissolved in water and which contains impurities such as by-products produced during the production of the (meth)acrylic acid. The crude (meth)acrylic acid solution refers to a solution containing a (meth)acrylic acid and impurities such as by-products produced during the production of the (meth)acrylic acid.

The production method of the present invention can sufficiently remove impurities in the easily-polymerizable compound-containing solution or the easily-polymerizable compound crystal-containing slurry.

### (Method for purifying easily-polymerizable compound)

The present invention also relates to a method for purifying an easily-polymerizable compound, the method including: forming easily-polymerizable compound crystals from an easily-polymerizable compound-containing solution or a mother liquor in an easily-polymerizable compound crystal-containing slurry using a crystal-forming apparatus including a scraper for scraping off easily-polymerizable compound crystals formed on an inner wall surface thereof and a shaft connected to the scraper, the apparatus further including a bushing on the shaft, the bushing being to be in contact with the solution or slurry, the bushing being made of an aromatic polyetherketone-containing material or the method including feeding an easily-polymerizable compound-containing solution or water from the outside of the apparatus to an area around a mechanical seal installed on the shaft.

The purification method of the present invention can efficiently purify an easily-polymerizable compound.

Preferred embodiments of the purification method of the present invention are the same as the preferred embodiments of the production method of the present invention described above.

### (Crystal-forming method)

The present invention also relates to a crystal-forming method, including: forming crystals from an easily-polymerizable compound-containing solution or a mother liquor in an easily-polymerizable compound crystal-containing slurry using a crystal-forming apparatus including a scraper for scraping off crystals formed on an inner wall surface thereof and a shaft connected to the scraper, the apparatus further including a bushing on the shaft, the bushing being to be in contact with the solution or slurry, the bushing being made of an aromatic polyetherketone-containing material or the method including feeding an easily-polymerizable compound-containing solution or water from the outside of the apparatus to an area around a mechanical seal installed on the shaft.

The crystals obtained by the crystal-forming method of the present invention may be easily-polymerizable compound crystals or may be a component other than the easily-polymerizable compound. In other words, the easily-polymerizable compound in the crystal-forming method of the present invention may not be a product, and may be a raw material, a by-product, a solvent, or the like in the solution or slurry. Preferred embodiments of the forming crystals are as described above. Also, preferred embodiments of the shaft bushing are as described above. The method of the present invention enables efficient purification of crystals.

### EXAMPLES

The present invention will be described in more detail below with reference to examples, but the present invention is not limited by the following examples, and appropriate modifications may be made within the scope that can conform to the gist of the above and later descriptions. All of them are included in the technical scope of the present invention.

Unless otherwise specified, "%" indicates "% by mass" and "parts" indicates "parts by mass."

### (Method for preparing acrylic acid aqueous solution)

An acrylic acid aqueous solution was prepared according to the method described in WO 2010/032665 as follows: propylene was catalytically oxidized in vapor phase to obtain an acrylic acid-containing gas; and the acrylic acid-containing gas was treated in an absorption column.

### (Crystal-forming apparatus)

As a crystal-forming apparatus, a purification apparatus was used which had the same (or partly the same) structure as that of the purification apparatus shown in FIG. 1, except that the apparatus included an intermediate bushing in addition to the bushings 1 and 1p and the following units:
the crystal-forming apparatus 100 having an inner diameter of 150 mm and a heat transfer area of 1.4 m²;
bushings (three) located in a part proximate to the power unit, an intermediate part, and a terminal part in the crystal-forming apparatus, wherein the bushing in the part proximate to the power unit and the bushing in the terminal part each have a length of 80 mm and the bushing in the intermediate part has a length of 50 mm, the distance between the bushings in the part proximate to the power unit and the intermediate part is 400 mm and the distance between the bushings in the intermediate part and the terminal part is 3000 mm, and all of the three bushings in the part proximate to the power unit, the intermediate part, and the terminal part are in contact (wetted) with the acrylic acid-containing solution or slurry containing acrylic acid crystals during operation of the crystal-forming apparatus;
the scraper 3 (material: PEEK, shown in FIG. 3(b)) for scraping off acrylic acid crystals formed on the inner wall surface;
the connection 4 (material: stainless steel) that is provided perpendicular to the longitudinal direction of the shaft;
the shaft 5 (material: stainless steel) connected to the scraper 3 via the connection 4; and
the mechanical seal 7 with a minimum distance to a bushing of 60 mm
The crystal-forming apparatus was operated under the following conditions.

### (Method for preparing feed slurry)

An acrylic acid aqueous solution was fed to the crystal-forming section 19 of the crystal-forming apparatus 100 from the feed port 9 for an easily-polymerizable compound (acrylic acid). A cooling medium was fed to a jacket provided around the wall of the crystal-forming apparatus for indirect cooling of the acrylic acid aqueous solution. The crystals formed and adhered to the inner wall surface of the crystal-forming apparatus 100 were scraped off with the scraper 3 provided in the crystal-forming apparatus. Thus, a slurry containing acrylic acid crystals was formed.

### (Example 1)

### (Operating conditions)

Rotation speed of shaft: 25 rpm
Amount of the slurry fed to the crystal-forming apparatus: 3,000 kg/h, feed temperature of the slurry: 8.0°C, crystal concentration of the slurry: 10.0% by mass
Flow rate of cooling medium: 11 m³/h, feed temperature of cooling medium : -6.1°C, composition of the mother liquor in the slurry: acrylic acid 92.7% by mass, acetic acid 2.5% by mass, water 3.1% by mass

The slurry containing acrylic acid crystals was fed to the crystal-forming section 19 of the crystal-forming apparatus 100 from the feed port 9 for an easily-polymerizable compound (acrylic acid). A cooling medium was fed to a jacket provided around the wall of the crystal-forming apparatus for indirect cooling of the mother liquor in the slurry containing acrylic acid crystals. The crystals formed and adhered to the inner wall surface of the crystal-forming apparatus 100 were scraped off with the scraper 3 provided in the crystal-forming apparatus. Thus, a slurry containing acrylic acid crystals was prepared.

Although a bushing made of PEEK was used, the crystal-forming apparatus stably operated for 50 hours without causing polymerization at the bushing induced by frictional heat. The conditions were as follows: the crystallization amount was 40 kg/h, the discharge port crystal concentration was 11.5% by mass, the temperature of the crystal-forming section (discharge temperature) was 7.9°C, the composition of the mother liquor in the slurry discharged from the crystal-forming section was acrylic acid 92.6% by mass, acetic acid 2.6% by mass, water 3.1% by mass.

### (Example 2)

A crystal-forming apparatus was operated as in Example 1, except that a bushing made of PTFE was used and water was fed at 0.5 L/h to an area around the mechanical seal 7. The apparatus stably operated for 50 hours.

### (Comparative Example 1)

A crystal-forming apparatus was operated as in Example 1, except that a bushing made of PTFE was used. After about five hours, polymerization was induced by frictional heat at or around the bushing and the polymer was caught in the mechanical seal in the apparatus, causing liquid leakage from the mechanical seal. Finally, the apparatus stopped.

### REFERENCE SIGNS LIST

1, 1p: bushing
3: scraper
4: connection
5: shaft
7: mechanical seal
9: feed port for easily-polymerizable compound-containing solution or easily-polymerizable compound crystal-containing slurry
11: discharge port for easily-polymerizable compound crystal-containing slurry
13: crystal-containing slurry
15: cooling-medium feed port
17: cooling-medium discharge port
19: crystal-forming section
21: power unit (motor)
23a: easily-polymerizable compound-containing solution or water
100: crystal-forming apparatus

## Claims

1. A heat exchanger configured to handle an easily-polymerizable compound-containing solution or an easily-polymerizable compound crystal-containing slurry,
the heat exchanger comprising a scraper for scraping off a substance adhered to an inner wall surface thereof, a shaft connected to the scraper, and a bushing on the shaft,
the bushing being to be in contact with the solution or slurry when the heat exchanger is used and being made of an aromatic polyetherketone-containing material.

2. The heat exchanger according to claim 1,
wherein the heat exchanger is a crystal-forming apparatus configured to form easily-polymerizable compound crystals from an easily-polymerizable compound-containing solution or a mother liquor in an easily-polymerizable compound crystal-containing slurry.

3. The heat exchanger according to claim 1 or 2,
wherein the scraper is made of an aromatic polyetherketone-containing material.

4. The heat exchanger according to any one of claims 1 to 3,
wherein at least one of the bushing or the scraper is made of a polyetheretherketone-containing material.

5. The heat exchanger according to any one of claims 1 to 4,
wherein the easily-polymerizable compound is a (meth)acrylic acid.

6. The heat exchanger according to any one of claims 1 to 5,
wherein the heat exchanger includes multiple bushings, with adjacent bushings being spaced at a distance of 5000 mm or less.

7. A crystal-forming apparatus configured to form easily-polymerizable compound crystals from an easily-polymerizable compound-containing solution or a mother liquor in an easily-polymerizable compound crystal-containing slurry,
the apparatus comprising a scraper for scraping off easily-polymerizable compound crystals formed on an inner wall surface thereof, a shaft connected to the scraper, and a bushing on the shaft,
the bushing being to be in contact with the solution or slurry when the apparatus is used and being made of an aromatic polyetherketone-containing material.

8. The crystal-forming apparatus according to claim 7,
wherein the scraper is made of an aromatic polyetherketone-containing material.

9. The crystal-forming apparatus according to claim 7 or 8,
wherein at least one of the bushing or the scraper is made of a polyetheretherketone-containing material.

10. The crystal-forming apparatus according to any one of claims 7 to 9,
wherein the easily-polymerizable compound is a (meth)acrylic acid.

11. The crystal-forming apparatus according to any one of claims 7 to 10,
wherein the apparatus includes multiple bushings, with adjacent bushings being spaced at a distance of 5000 mm or less.

12. A purification apparatus comprising:
the heat exchanger according to any one of claims 1 to 6; or
the crystal-forming apparatus according to any one of claims 7 to 11.

13. A crystal-forming method, comprising:
forming crystals from an easily-polymerizable compound-containing solution or a mother liquor in an easily-polymerizable compound crystal-containing slurry using a crystal-forming apparatus comprising a scraper for scraping off crystals formed on an inner wall surface thereof and a shaft connected to the scraper,
the apparatus further comprising a bushing on the shaft, the bushing being to be in contact with the solution or slurry,
the bushing being made of an aromatic polyetherketone-containing material or the method comprising feeding an easily-polymerizable compound-containing solution or water from the outside of the apparatus to an area around a mechanical seal installed on the shaft.

14. The crystal-forming method according to claim 13,
wherein the aromatic polyetherketone is polyetheretherketone.

15. The crystal-forming method according to claim 13 or 14,
wherein the heat exchanger comprises a crystal-forming section configured to form easily-polymerizable compound crystals from an easily-polymerizable compound-containing solution or a mother liquor in an easily-polymerizable compound crystal-containing slurry, the solution or the mother liquor in the slurry in the crystal-forming section has a melting point higher than the melting point of the easily-polymerizable compound-containing solution or water used in the feeding.

16. The crystal-forming method according to any one of claims 13 to 15,
wherein in the heat exchanger, the crystal-forming section configured to form easily-polymerizable compound crystals from an easily-polymerizable compound-containing solution or a mother liquor in an easily-polymerizable compound crystal-containing slurry has a temperature lower than the melting point of a pure substance of the easily-polymerizable compound by 1°C to 15°C.

17. The crystal-forming method according to any one of claims 13 to 16,
wherein in the forming crystals, the shaft is rotated at a rotation speed of 1 to 80 rpm.

18. The crystal-forming method according to any one of claims 13 to 17,
wherein the easily-polymerizable compound is a (meth)acrylic acid.

19. A method for producing an easily-polymerizable compound, the method comprising forming easily-polymerizable compound crystals from an easily-polymerizable compound-containing solution or a mother liquor in an easily-polymerizable compound crystal-containing slurry using a crystal-forming apparatus comprising a scraper for scraping off easily-polymerizable compound crystals formed on an inner wall surface thereof and a shaft connected to the scraper,
the apparatus further comprising a bushing on the shaft, the bushing being to be in contact with the solution or slurry,
the bushing being made of an aromatic polyetherketone-containing material or the method comprising feeding an easily-polymerizable compound-containing solution or water from the outside of the apparatus to an area around a mechanical seal installed on the shaft.

20. The method for producing an easily-polymerizable compound according to claim 19,
wherein the aromatic polyetherketone is polyetheretherketone.

21. The method for producing an easily-polymerizable compound according to claim 19 or 20,
wherein the apparatus comprises a crystal-forming section configured to form easily-polymerizable compound crystals from an easily-polymerizable compound-containing solution or a mother liquor in an easily-polymerizable compound crystal-containing slurry, the solution or the mother liquor in the slurry in the crystal-forming section has a melting point higher than the melting point of the easily-polymerizable compound-containing solution or water used in the feeding.

22. The method for producing an easily-polymerizable compound according to any one of claims 19 to 21,
wherein in the apparatus, the crystal-forming section configured to form easily-polymerizable compound crystals from an easily-polymerizable compound-containing solution or a mother liquor in an easily-polymerizable compound crystal-containing slurry has a temperature lower than the melting point of a pure substance of the easily-polymerizable compound by 1°C to 15°C.

23. The method for producing an easily-polymerizable compound according to any one of claims 19 to 22,
wherein in the forming crystals, the shaft is rotated at a rotation speed of 1 to 80 rpm.

24. The method for producing an easily-polymerizable compound according to any one of claims 19 to 23,
wherein the easily-polymerizable compound is a (meth)acrylic acid.

25. The method for producing an easily-polymerizable compound according to claim 24,
wherein the (meth)acrylic acid is produced from at least one raw material selected from the group consisting of propane, propylene, acrolein, isobutene, methacrolein, acetic acid, lactic acid, isopropanol, 1,3-propanediol, glycerol, and 3-hydroxypropionic acid.

26. A method for purifying an easily-polymerizable compound, the method comprising:
forming easily-polymerizable compound crystals from an easily-polymerizable compound-containing solution or a mother liquor in an easily-polymerizable compound crystal-containing slurry using a crystal-forming apparatus comprising a scraper for scraping off easily-polymerizable compound crystals formed on an inner wall surface thereof and a shaft connected to the scraper,
the apparatus further comprising a bushing on the shaft, the bushing being to be in contact with the solution or slurry,
the bushing being made of an aromatic polyetherketone-containing material or the method comprising feeding an easily-polymerizable compound-containing solution or water from the outside of the apparatus to an area around a mechanical seal installed on the shaft.
